# FASCICULE DE BREVET EUROPEEN

(11) **EP 3 019 163 B1**
(45) Date de publication et mention de la délivrance du brevet: **03.07.2019**
(21) Numéro de dépôt: 14747081.9
(22) Date de dépôt: 03.07.2014
(51) Int. Cl.: A61K 31/13, A61K 33/00, A61K 45/06, A61P 25/16, A61P 25/28

(54) **ASSOCIATION DE XÉNON ET D'UN ANTAGONISTE DES RÉCEPTEURS NMDA POUR LUTTER CONTRE UNE MALADIE NEURODÉGÉNÉRATIVE**
KOMBINATION VON XENON MIT EINEM NMDA-REZEPTOR-ANTAGONISTEN ZUR BEKÄMPFUNG EINER NEURODEGENERATIVEN KRANKHEIT
COMBINATION OF XENON WITH AN NMDA RECEPTOR ANTAGONIST FOR FIGHTING A NEURODEGENERATIVE DISEASE

(30) Priorité: 08.07.2013 FR 1356688
(43) Date de publication de la demande: 18.05.2016
(73) Titulaire: L'Air Liquide Société Anonyme pour l'Etude et l'Exploitation des Procédés Georges Claude, 75007 Paris (FR); L'Institut du Cerveau et de la Moelle Epiniere, 75013 Paris (FR)
(72) Inventeur: MICHEL, Patrick, F-75004 Paris (FR); LAVAUR, Jérémie, F-75007 Paris (FR); HIRSCH, Etienne, F-78000 Versailles (FR); LEMAIRE, Marc, F-75014 Paris (FR); PYPE, Jan, B-1540 Herne (BE)
(74) Mandataire: Pittis, Olivier
(86) Numéro de dépôt international: PCT/FR2014/051710
(87) Numéro de publication internationale: WO 2015/004371

(56) Documents cités:
- WO-A2-2005/067945
- DAVID ET AL: "Nitrous Oxide and Xenon Prevent Amphetamine-Induced Carrier-Mediated Dopamine Release in a Memantine-Like Fashion and Protect Against Behavioral Sensitization", BIOLOGICAL PSYCHIATRY, ELSEVIER SCIENCE, NEW YORK, NY; US, vol. 60, no. 1, 1 juillet 2006 (2006-07-01), pages 49-57, XP005512613, ISSN: 0006-3223, DOI: 10.1016/J.BIOPSYCH.2005.10.007
- CHEN H-S V ET AL: "The chemical biology of clinically tolerated NMDA receptor antagonists", JOURNAL OF NEUROCHEMISTRY, WILEY INTERSCIENCE, NEW YORK, NY, US, vol. 97, no. 6, 19 juin 2006 (2006-06-19), pages 1611-1626, XP002418439, ISSN: 0022-3042, DOI: 10.1111/J.1471-4159.2006.03991.X
- SEEMAN P ET AL: "Memantine agonist action at dopamine D2High receptors", SYNAPSE, WILEY AND SONS, CHICHESTER, GB, vol. 62, no. 2, 1 février 2008 (2008-02-01), pages 149-153, XP002657695, ISSN: 0887-4476, DOI: 10.1002/SYN.20472 [extrait le 2007-11-13]
- PARSONS ET AL: "Memantine: a NMDA receptor antagonist that improves memory by restoration of homeostasis in the glutamatergic system - too little activation is bad, too much is even worse", NEUROPHARMACOLOGY, PERGAMON PRESS, OXFORD, GB, vol. 53, no. 6, 9 octobre 2007 (2007-10-09), pages 699-723, XP022293457, ISSN: 0028-3908, DOI: 10.1016/J.NEUROPHARM.2007.07.013
- S. E. KOTERMANSKI ET AL: "Mg2+ Imparts NMDA Receptor Subtype Selectivity to the Alzheimer's Drug Memantine", JOURNAL OF NEUROSCIENCE, vol. 29, no. 9, 4 mars 2009 (2009-03-04), pages 2774-2779, XP055094319, ISSN: 0270-6474, DOI: 10.1523/JNEUROSCI.3703-08.2009

## Description

L'invention porte sur l'utilisation de xénon gazeux en tant que médicament inhalable, utilisé en combinaison avec un antagoniste des récepteurs NMDA, à savoir la mémantine ou la nitromémantine, pour traiter, ralentir ou prévenir une dégradation neurologique, en rapport avec, ou consécutive à une maladie neurodégénérative, en particulier la maladie d'Alzheimer ou de Parkinson.

Les récepteurs/canaux NMDA (pour N-méthyl-D-aspartate) sont des entités moléculaires de la membrane plasmique des cellules neuronales. Ces récepteurs, sont la cible des molécules de glutamate libérées dans l'espace synaptique et extra-synaptique, le glutamate étant un neurotransmetteur excitateur assurant la communication d'une cellule nerveuse à une autre.

Dans les maladies neurodégénératives, comme la maladie d'Alzheimer, les cellules nerveuses produisant du glutamate, le libèrent en quantités anormalement élevées, provoquant une excitotoxicité neuronale résultant d'une stimulation excessive des récepteurs NMDA. Cette excitotoxicité suspectée d'être partie prenante de la maladie d'Alzheimer (Hynd et coll., Neurochem Int, 2004) et aussi d'autres pathologies dégénératives comme la maladie de Parkinson (Metha et coll., Eur J Pharmacol, 2013), engendre des conséquences néfastes pour les neurones post-synaptiques portant les récepteurs NMDA, suite notamment à une entrée trop importante d'ions calcium (Ca²⁺) dans le compartiment intracellulaire.

La mémantine, un antagoniste non-compétitif et de faible affinité du récepteur NMDA, est utilisée pour lutter contre la maladie d'Alzheimer. La mémantine interagit directement avec les récepteurs NMDA, en les bloquant, et en limitant l'entrée d'ions Ca²⁺, réduisant ainsi la toxicité due à l'excès de glutamate. Il en résulte une amélioration de la transmission des messages nerveux entre les cellules neuronales et un ralentissement du déclin de la mémoire et de la cognition dans le cadre de la maladie d'Alzheimer. Il est fait référence à Chen H-S. V. et al., Journal of Neurochemistry 97 (2006), Seeman P. et al., Synapse 62 (2008) et Parsons et al., Neuropharmacology 53 (2007).

Cependant, l'effet de la mémantine est limité et cette molécule n'est pas dénuée d'effets indésirables, tel que confusion, vertiges, somnolence, céphalées, insomnies, agitation, hallucinations, vomissements, anxiété, ...

WO 2005/067945 divulgue l'utilisation d'un mélange de xénon/monoxyde de carbone pour le traitement des maladies neurodégénératives comme la maladie d'Alzheimer et la maladie de Parkinson.

Le problème est donc de proposer un traitement amélioré, permettant d'éviter, ralentir ou minimiser toute dégradation neurologique en rapport avec, ou consécutive à une maladie neurodégénérative de type maladie d'Alzheimer ou de Parkinson, tout en diminuant les doses de mémantine utilisées et donc les effets indésirables négatifs liés à l'utilisation de ce composé.

La solution, selon la présente invention, est un médicament gazeux contenant du xénon pour une utilisation par inhalation, en association avec au moins un antagoniste des récepteurs NMDA sous forme liquide ou solide, pour traiter, ralentir ou prévenir une dégradation neurologique consécutive à une maladie neurodégénérative chez un patient humain.

En effet, dans le cadre de la présente invention, il a été mis en évidence que l'association de xénon et d'un antagoniste des récepteurs NMDA, telle la mémantine ou un dérivé ou un composé de la mémantine, en particulier la nitromémantine, conduit à une action synergique de ces composés et qu'une telle association peut constituer un traitement prometteur des dégradations neurologiques résultant de maladies neuro-dégénératives, telle la maladie d'Alzheimer, notamment.

Une telle association repose notamment sur les modes d'actions de ces composés.

Ainsi, le xénon possède des propriétés inhibitrices des voies de signalisation glutamatergiques excitatrices, via son action antagoniste sur les récepteurs NMDA, mais aussi sur les récepteurs α-amino-3-hydroxy-5-méthylisoazol-4-propionate (AMPA), ainsi que sur les récepteurs kainate, qui composent les récepteurs ionotropiques au glutamate.

Dès lors, l'association xénon/mémantine ou xénon/nitromémantine conduit à une synergie d'action au niveau des récepteurs glutamatergiques et notamment NMDA, sans risquer d'augmenter les effets indésirables de la mémantine.

En d'autres termes, l'ajout de xénon permet de renforcer les effets bénéfiques de la mémantine ou de la nitromémantine par un effet synergique mais sans engendrer les effets indésirables de la mémantine ou de la nitromémantine.

Selon le cas, le médicament gazeux selon l'invention peut comprendre l'une ou plusieurs des caractéristiques suivantes :
- l'antagoniste des récepteurs NMDA est sous forme solide, en particulier sous forme de comprimé ou de gélule.
- l'antagoniste des récepteurs NMDA est la mémantine, ou un dérivé ou un composé de la mémantine.
- l'antagoniste des récepteurs NMDA est la mémantine ou la nitromémantine.
- la maladie neuro-dégénérative est la maladie d'Alzheimer ou de Parkinson, de préférence la maladie d'Alzheimer.
- la dose journalière d'antagoniste des récepteurs NMDA administrée au patient est inférieure ou égale à 20 mg/jour.
- le xénon gazeux est administré au patient avant, concomitamment ou après administration de l'antagoniste des récepteurs NMDA, de préférence après administration de l'antagoniste des récepteurs NMDA.
- la dégradation neurologique comprend une entrée excessive d'ions Ca²⁺ dans une ou plusieurs populations de neurones vulnérables du patient.
- il contient en outre de l'oxygène, de préférence au moins 21% en volume d'oxygène.
- il est constitué de xénon et d'oxygène.
- il est constitué de xénon, d'azote et d'oxygène.
- le patient est un homme ou une femme.
- le patient est un être humain âgé de plus de 30 ans, de préférence de plus de 40 ans, en particulier de plus de 50 ans.
- le médicament contient une quantité efficace de xénon.
- le médicament contient une quantité non anesthésique de xénon, i.e. sub-anesthésique.
- il contient entre 10 et 80% en volume de xénon.
- le médicament contient entre 15 et 80% en volume de xénon.
- le médicament contient au moins 20% en volume de xénon.
- le médicament contient moins de 60% en volume de xénon.
- le médicament moins de 75% en volume de xénon.
- le médicament contient entre 20 et 50% en volume de xénon.
- le médicament contient entre 20 et 40% en volume de xénon.
- le xénon est mélangé avec de l'oxygène juste avant ou au moment de son inhalation par le patient ou se présente sous forme d'un mélange gazeux « prêt à l'emploi » en pré-mélange avec de l'oxygène, et contient éventuellement un autre composé gazeux, par exemple de l'azote.
- le médicament est constitué d'un mélange gazeux formé d'oxygène et d'azote.
- le gaz est administré au patient, une ou plusieurs fois par jour.
- le gaz est administré au patient pendant une durée d'inhalation de quelques minutes à quelques heures, typiquement entre 15 minutes et 6 heures, préférentiellement moins de 4 heures.
- la durée, la posologie et la fréquence d'administration sont fonction de l'évolution de l'état neurologique du patient considéré et seront préférentiellement fixées par le médecin ou personnel soignant en fonction de l'état neurologique du patient considéré.
- le médicament gazeux est conditionné dans une bouteille de gaz ayant une contenance (équivalent en eau) allant jusqu'à 50 litres, typiquement de l'ordre de 0,5 à 15 litres et/ou à une pression inférieure ou égale à 350 bar absolus, typiquement une pression comprise entre 2 et 300 bar.
- le médicament gazeux est conditionné dans une bouteille de gaz équipée d'un robinet ou d'un robinet à détendeur intégré permettant de contrôler le débit et éventuellement la pression du gaz délivré.
- le médicament gazeux est conditionné dans une bouteille de gaz en acier, en aluminium ou en matériaux composites.
- lors du traitement, l'administration du médicament gazeux au patient se fait par inhalation au moyen d'un masque facial ou nasal, de lunettes nasales ou au travers de tout autre système d'administration d'un gaz inhalable.

Dit autrement, l'invention concerne un médicament gazeux contenant du xénon pour une utilisation par inhalation en association avec au moins un antagoniste des récepteurs NMDA sous forme liquide ou solide, pour traiter, ralentir ou prévenir une dégradation neurologique consécutive à une maladie neuro-dégénérative chez un patient humain.

L'invention trouve utilité pour traiter, ralentir ou prévenir au moins une dégradation neurologique consécutive à une maladie neurodégénérative chez un patient humain, dans laquelle :
i) on identifie un patient humain atteint d'une maladie neurodégénérative ou susceptible d'être atteint d'une telle maladie neurodégénérative,
ii) on administre par inhalation, audit patient, un médicament gazeux contenant du xénon, et
iii) on administre, audit patient, au moins un antagoniste des récepteurs NMDA sous forme liquide ou solide,
de manière à permettre une protection neuronale et ainsi traiter, ralentir ou prévenir au moins une dégradation neurologique résultant de la maladie neurodégénérative chez ledit patient.

De préférence, à l'étape i) :
- le patient humain est un homme ou une femme.
- le patient humain est âgé de plus de 30 ans, de préférence de plus de 40 ans, en particulier de plus de 50 ans.
- l'identification du patient se fait par un médecin ou analogue.
- l'identification du patient se fait par examen technique de dépistage.
- la maladie neurodégénérative susceptible d'engendrer au moins une dégradation neurologique est la maladie d'Alzheimer ou de Parkinson, de préférence la maladie d'Alzheimer.
- ladite dégradation neurologique comprend une entrée excessive d'ions Ca²⁺ dans une ou plusieurs populations de neurones vulnérables du patient.

De préférence, à l'étape ii) :
- on administre, audit patient, au moins un antagoniste des récepteurs NMDA sous forme solide.
- on administre au moins un antagoniste des récepteurs NMDA par voie entérale, i.e. la voie orale.
- on administre, audit patient, au moins un antagoniste des récepteurs NMDA sous forme de comprimé ou de gélule.
- on administre au patient de la mémantine, ou un dérivé ou un composé de la mémantine en tant qu'antagoniste des récepteurs NMDA.
- on administre au patient de la mémantine ou de la nitromémantine en tant qu'antagoniste des récepteurs NMDA.
- on administre au patient une dose journalière d'antagoniste des récepteurs NMDA inférieure ou égale à 20 mg/jour.
- on administre, audit patient, au moins un antagoniste des récepteurs NMDA avant, concomitamment ou après inhalation de xénon par le patient.

De préférence, à l'étape iii) :
- la durée, la posologie et la fréquence d'administration du xénon sont choisies et/ou fixées en fonction de l'évolution de l'état neurologique du patient considéré.
- on administre une quantité efficace de xénon.
- on administre une quantité non anesthésique de xénon.
- on administre de 10 à 80% en volume de xénon, de préférence entre 20 et 50% en volume de xénon.
- on mélange le xénon avec de l'oxygène avant ou au moment de son inhalation par le patient, de préférence avec au moins 21% en volume d'oxygène.
- on administre un mélange gazeux prêt à l'emploi constitué de xénon et d'oxygène (mélange binaire) ou de xénon, d'oxygène et d'azote (mélange ternaire).
- on administre le xénon gazeux une ou plusieurs fois par jour au patient.
- on administre le xénon gazeux au patient pendant une durée d'inhalation de quelques minutes à quelques heures, typiquement entre 15 minutes et 6 heures, préférentiellement moins de 4 heures.
- on administre le xénon gazeux au moyen d'un masque facial ou nasal, de lunettes nasales ou au travers de tout autre système ou dispositif d'administration de gaz à un patient.

### Exemple

Afin de démontrer l'efficacité de la combinaison du xénon et d'un antagoniste des récepteurs NMDA selon la présente invention, nous avons mis en place un modèle cellulaire de neurones corticaux dans lequel la mort neuronale est déclenchée en bloquant les systèmes de recapture au glutamate grâce à un traitement prolongé avec du L-trans-pyrrolidine-2,4-dicarboxylic acid (PDC), un composé dont le mécanisme d'action a été décrit précédemment parZuiderwijk et coll, (Europ J Pharmacol, 1994).

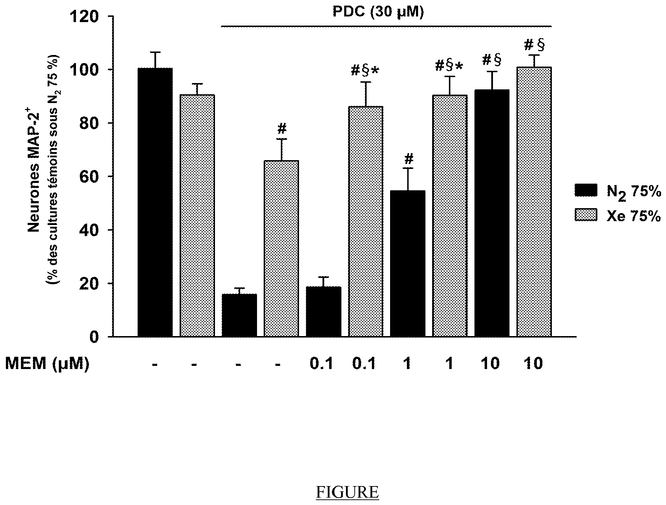

La technique mise en oeuvre est décrite ci-dessous et les résultats obtenus sont illustrés sur la Figure ci-annexée montrant les effets protecteurs synergiques du xénon et de la mémantine dans un modèle cellulaire mimant une dégénérescence corticale chronique.

### Protocole d'obtention des cultures primaires de cortex

Des cultures sont préparées à partir de cortex d'embryons de rats, prélevés sur des rates Wistar, au jour 15,5 de gestation.

Le procédé d'obtention des cultures de cortex comprend l'obtention d'une suspension cellulaire homogène par dissociation mécanique, c'est-à-dire non enzymatique, du tissu embryonnaire, en utilisant du milieu L15 de Leibovitz (Sigma Aldrich).

Des aliquotes de cette suspension sont ajoutés à des plaques Nunc de 48 puits, qui ont été préalablement revêtues dune fine couche de polyéthylènimine (1 mg/ml, tampon borate pH 8,3) pour permettre l'adhésion des cellules neuronales (cf. Toulorge et coll, Faseb J, 2011).

La densité d'ensemencement est comprise entre environ 20000 et 30000 cellules/cm².

Les cultures de cortex sont maintenues dans du milieu de culture Neurobasal, contenant un cocktail B27 sans antioxydant, du supplément N2, de la glutamine (2 mM) et un cocktail pénicilline/streptomycine (cf. Nafia et coll, J Neurochem, 2008). Le milieu et le supplément sont disponibles auprès de Life Technologies.

Six heures après la mise en culture, on ajoute 0,5 µM d'antimitotique cytarabine (Sigma Aldrich) afin de limiter la prolifération astrocytaire.

Jusqu'au moment où l'on évalue les effets des gaz d'intérêt, les cultures sont placées dans une enceinte conventionnelle thermostatée à 37°C, dans laquelle le CO₂ est maintenu à 5% en volume et où l'atmosphère est saturée en eau.

Aucun changement de milieu de culture n'est effectué pendant toute la période de la culture.

### Traitements pharmacologiques des cultures

Le processus dégénératif est déclenché par application d'un bloqueur des systèmes de recapture du glutamate, le PDC (Zuiderwijk et al, Europ JPharmacol, 1994).

Le bloqueur des récepteurs NMDA, la mémantine est ajoutée aux cultures avant l'application de PDC. Ces deux produits (PDC et mémantine) proviennent de chez RD Systems.

### Maintenance des cultures sous atmosphère gazeuse contrôlée

Une fois les traitements pharmacologiques effectués, les boîtes multi-puits contenant les cellules en culture et la boite servant à l'humidification du compartiment interne de la chambre, sont placées sur une embase métallique qui reçoit la chambre d'incubation en Plexiglas. Les deux pièces (embase et chambre en Plexiglas) sont réunies par vissage, de manière jointive.

On injecte ensuite un mélange gazeux d'intérêt comprenant (% en volume) : 20 % d'O₂, 5 % de CO₂ et 75 % de gaz testé, dans la chambre d'incubation, avec valves d'entrée et de sortie ouvertes, tout en contrôlant le débit de sortie grâce à un débitmètre. Les gaz testés sont de l'azote et du xénon.

Le débit de sortie de référence, fixé pour l'air à 10 litres/mn, est corrigé en fonction de la densité du mélange utilisé.

Lorsque la mesure du CO₂, atteint 5 % en sortie, on stoppe l'injection du mélange gazeux et la chambre est rendue totalement étanche en fermant les valves d'entrée et de sortie.

La chambre d'exposition est alors placée dans une enceinte à 37°C pendant toute la durée du protocole expérimental.

### Immunodétection de la protéine « microtubule-associated protein-2 » (MAP-2) et comptages cellulaires

Après rupture de l'étanchéité par ouverture des valves d'entrée et de sortie et dévissage de la chambre de son embase, les cultures sont fixées avec 4% de formaldéhyde dans du PBS pendant 12 mn et ensuite incubées à 4°C avec un anticorps monoclonal anti-MAP-2 (dilution 1:200; clone AP-20; Sigma-Aldrich) pendant 3 jours.

La révélation de cet anticorps est obtenue avec un anticorps secondaire anti-souris couplé à la cyanine-3 (Sigma Aldrich ; dilution 1:1000 dans du PBS).

L'acquisition des images est réalisée à l'objectif 10x en utilisant un microscope inverséNikon TE-300, équipé d'une caméra digitale refroidie ORCA-ER et d'un logiciel d'acquisition d'images HCI (Hamamatsu).

Les résultats obtenus dans le modèle de dégénérescence corticale par traitement chronique au PDC, révèlent des effets protecteurs synergiques entre le xénon et la mémantine qui sont résumés dans le Tableau suivant et représentés sur la Figure.

**Tableau : Résumé des principaux résultats de l'étude in vitro sur les cultures de cortex**

| Cultures de cortex à J 16 | | |
|---|---|---|
| Traitements J 12 - J 16 | Mélange gazeux (20% O₂ + 5% CO₂ + 75 % gaz testé) ; % en volume | Survie neuronale |
| Groupe témoin I | N₂ | +++ |
| Groupe témoin II | Xe | +++ |
| Groupe PDC (30µM) | N₂ | - |
| Groupe PDC (30 µM) | Xe | ++ |
| Groupe PDC (30 µM) + mémantine (0,1 µM) | N₂ | - |
| Groupe PDC (30 µM) + mémantine (0,1 µM) | Xe | +++ |
| Groupe PDC (30 µM) + mémantine (1 µM) | N₂ | ++ |
| Groupe PDC (30 µM) + mémantine (1 µM) | Xe | +++ |
| Groupe PDC (30 µM) + mémantine (10 µM) | N₂ | +++ |
| Groupe PDC (30 µM) + mémantine (10 µM) | Xe | +++ |

Dans le Tableau précédent, une réponse favorable, synonyme d'une baisse du niveau de mort des cellules neuronales en présence des traitements d'intérêt, est désignée par un signe « + », « ++ », ou « +++ » (+++ = niveau de référence).A l'inverse, une réponse défavorable est représentée par un signe « - », synonyme d'une augmentation de la mort neuronale.

La mémantine agit en bloquant les récepteurs NMDA et le xénon en bloquant ces mêmes récepteurs mais probablement via un mécanisme distinct, impliquant le blocage du site de fixation de la glycine, un acide aminé qui agit comme coactivateur du récepteur.

La cytarabine ou Ara-C est appliquée initialement à tous les puits de cultures afin de limiter la prolifération des astrocytes. Le PDC, induit quant à lui, un processus dégénératif en prévenant la recapture du glutamate produit et libéré de manière endogène par les cellules neuronales en culture.

Au vu des résultats illustrés en Figure annexée, on constate que la combinaison du xénon et d'un antagoniste des récepteurs NMDA, à savoir ici la mémantine, conduit à un effet neuroprotecteur significativement supérieur à l'effet de chaque molécule, prise séparément.

En fait, une réelle synergie d'action de l'association xénon/mémantine se met en place.

Tout d'abord, ces essais ont montré que le blocage des systèmes de recapture du glutamate par le PDC cause une perte d'environ 85 % des cellules neuronales corticales, à une concentration de 30 µM, sous une atmosphère contenant 75 vol.% d'azote.

Cependant, les effets délétères du PDC sont partiellement prévenus lorsque l'on remplace l'azote par le xénon puisque que le taux de survie passe respectivement, de 15,9 % à 65,9 %.

De manière intéressante, la mémantine n'a pas d'effet significatif dans ce modèle cellulaire lorsqu'elle est appliquée seule à 0,1 µM, sous atmosphère contenant 75 % d'azote. Cependant, elle potentialise les effets protecteurs obtenus sous 75 % de xénon, permettant d'atteindre un taux de survie supérieur à 86,1 % dans ces conditions, contre environ 65,9% en l'absence de mémantine.

La potentialisation des effets du xénon est aussi observée lorsqu'on applique une concentration de mémantine de 1 µM qui permet d'atteindre, à elle seule, un taux de survie de 54, 6%.

Enfin, à 10 µM de mémantine, la synergie n'est plus observée, étant donné que la mémantine exerce déjà, en soi, un effet protecteur qui permet le sauvetage de plus de 90 % des cellules neuronales.

Les résultats décrits sur la Figure ci-annexée illustrent les effets protecteurs synergiques du xénon et de la mémantine dans un modèle cellulaire mimant une dégénérescence corticale chronique.

Ces résultats ont été obtenus sur des cultures de cortex de rat qui ont été traitées à partir du jour 12 *in vitro* et pendant les 4 jours suivants, avec un inhibiteur de recapture du glutamate, le PDC (30 µM) sous atmosphère contenant 75 % d'azote (N₂ 75) ou 75 % de xénon (Xe 75), en présence ou non de mémantine (MEM), testée à 0.1, 1 ou 10 µM.

Les cultures sont ensuite récupérées pour fixation et analyse. La survie neuronale est quantifiée dans les différentes conditions expérimentales testées, en comptant le nombre de corps cellulaires immuno-positifs pour le marqueur pan-neuronal MAP-2.

Les résultats sont exprimés en % des cultures non-traitées au PDC, maintenues sous 75 % d'azote (condition témoin).

Ainsi, l'étude statistique faite par analyse de variance à un facteur ou méthode One-way ANOVA (ANalysis Of VAriance), suivie d'un test de Student-Newman-Keuls (n= 6 pour chaque point expérimental) démontre que :
- le xénon seul (sans mémantine) produit un effet significativement supérieur à celui de la condition azote seul (^{#} p<0.05, augmenté par rapport aux cultures traitées avec 30 µM de PDC sous 75 % d'azote).
- le xénon a un effet synergique avec la mémantine, significativement supérieur à la condition xénon, seul (^{§} p<0.05, augmenté par rapport aux cultures traitées avec 30 µM de PDC sous 75 % de xénon) et à la condition 75 % d'azote, même en présence de mémantine aux concentrations de 0.1 et 1 µM (* p<0.05, augmenté par rapport aux cultures traitées avec 30 µM de PDC sous 75 % d'azote, en présence d'une même concentration de mémantine).
Le xénon conduit donc, lorsqu'il est associé à un antagoniste des récepteurs NMDA, telle la mémantine ou la nitromémantine en tant qu'antagoniste des récepteurs NMDA, à un effet synergique dans le traitement, le ralentissement ou la prévention des dégradations neurologiques consécutives à une maladie neurodégénérative, en particulier la maladie d'Alzheimer.

## Revendications

1. Médicament gazeux contenant du xénon pour une utilisation par inhalation en association avec au moins un antagoniste des récepteurs NMDA sous forme liquide ou solide, pour traiter, ralentir ou prévenir une dégradation neurologique consécutive à une maladie neuro-dégénérative chez un patient humain.

2. Médicament pour une utilisation selon la revendication précédente, **caractérisé en ce qu'**il contient entre 10 et 80% en volume de xénon.

3. Médicament pour une utilisation selon l'une des revendications précédentes, **caractérisé en ce que** l'antagoniste des récepteurs NMDA sous forme solide, en particulier sous forme de comprimé ou de gélule.

4. Médicament pour une utilisation selon l'une des revendications précédentes, **caractérisé en ce que** l'antagoniste des récepteurs NMDA est la mémantine ou la nitromémantine.

5. Médicament pour une utilisation selon l'une des revendications précédentes, **caractérisé en ce que** la maladie neurodégénérative est la maladie d'Alzheimer ou de Parkinson, de préférence la maladie d'Alzheimer.

6. Médicament pour une utilisation selon l'une des revendications précédentes, **caractérisé en ce que** la dose journalière d'antagoniste des récepteurs NMDA administrée au patient est inférieure ou égale à 20 mg/jour.

7. Médicament pour une utilisation selon l'une des revendications précédentes, **caractérisé en ce que** le xénon gazeux est administré au patient avant, concomitamment ou après administration de l'antagoniste des récepteurs NMDA, de préférence après administration de l'antagoniste des récepteurs NMDA.

8. Médicament pour une utilisation selon l'une des revendications précédentes, **caractérisé en ce que** la dégradation neurologique comprend une entrée excessive d'ions Ca²⁺ dans une ou plusieurs populations de neurones vulnérables du patient.

9. Médicament pour une utilisation selon l'une des revendications précédentes, **caractérisé en ce qu'**il contient en outre de l'oxygène, de préférence au moins 21% en volume d'oxygène, de préférence il est constitué de xénon et d'oxygène ou de xénon, d'azote et d'oxygène.

10. Médicament pour une utilisation selon l'une des revendications précédentes, **caractérisé en ce que** le patient est un être humain âgé de plus de 30 ans, en particulier âgé de plus de 50 ans.

11. Médicament pour une utilisation selon l'une des revendications précédentes, **caractérisé en ce qu'**il contient au moins 20% en volume de xénon et/ou il contient moins de 75% en volume de xénon, de préférence moins de 60% en volume de xénon.

12. Médicament pour une utilisation selon l'une des revendications précédentes, **caractérisé en ce qu'**il est administré au patient, une ou plusieurs fois par jour.

13. Médicament pour une utilisation selon l'une des revendications précédentes, **caractérisé en ce qu'**il est administré au patient pendant une durée d'inhalation de quelques minutes à quelques heures, en particulier entre 15 minutes et 4 heures.

## Patentansprüche

1. Gasförmiges Medikament, enthaltend Xenon für eine Verwendung durch Inhalation in Verbindung mit mindestens einem NMDA-Rezeptor-Antagonisten in flüssiger oder fester Form, um eine neurologische Schädigung als Folge einer neurodegenerativen Krankheit bei einem menschlichen Patienten zu behandeln, zu verlangsamen oder ihr vorzubeugen.

2. Medikament für eine Verwendung nach dem vorstehenden Anspruch, **dadurch gekennzeichnet, dass** es zwischen 10 und 80 Vol.-% Xenon enthält.

3. Medikament für eine Verwendung nach einem der vorstehenden Ansprüche, **dadurch gekennzeichnet, dass** der NMDA-Rezeptor-Antagonist in fester Form, insbesondere in Tabletten- oder Kapselform.

4. Medikament für eine Verwendung nach einem der vorstehenden Ansprüche, **dadurch gekennzeichnet, dass** der NMDA-Rezeptor-Antagonist Memantin oder Nitromemantin ist.

5. Medikament für eine Verwendung nach einem der vorstehenden Ansprüche, **dadurch gekennzeichnet, dass** die neurodegenerative Krankheit die Alzheimer'sche oder Parkinson-Krankheit ist, vorzugsweise die Alzheimer'sche Krankheit.

6. Medikament für eine Verwendung nach einem der vorstehenden Ansprüche, **dadurch gekennzeichnet, dass** die tägliche Dosis NMDA-Rezeptor-Antagonist, die dem Patienten verabreicht wird, kleiner oder gleich 20 mg/Tag ist.

7. Medikament für eine Verwendung nach einem der vorstehenden Ansprüche, **dadurch gekennzeichnet, dass** das gasförmige Xenon dem Patienten vor, gleichzeitig mit oder nach Verabreichung des NMDA-Rezeptor-Antagonisten, vorzugsweise nach Verabreichung des NMDA-Rezeptor-Antagonisten, verabreicht wird.

8. Medikament für eine Verwendung nach einem der vorstehenden Ansprüche, **dadurch gekennzeichnet, dass** die neurodegenerative Störung einen übermäßigen Eintritt von Ca²⁺-Ionen in eine oder mehrere verwundbare Neuronenpopulationen des Patienten umfasst.

9. Medikament für eine Verwendung nach einem der vorstehenden Ansprüche, **dadurch gekennzeichnet, dass** es weiter Sauerstoff enthält, vorzugsweise mindestens 21 Vol.-% Sauerstoff, es vorzugsweise aus Xenon und Sauerstoff oder aus Xenon, Stickstoff und Sauerstoff besteht.

10. Medikament für eine Verwendung nach einem der vorstehenden Ansprüche, **dadurch gekennzeichnet, dass** der Patient ein Mensch im Alter von mehr als 30 Jahren ist, insbesondere im Alter von mehr als 50 Jahren.

11. Medikament für eine Verwendung nach einem der vorstehenden Ansprüche, **dadurch gekennzeichnet, dass** es mindestens 20 Vol.-% Xenon enthält und/oder es weniger als 75 Vol.-% Xenon enthält, vorzugsweise weniger als 60 Vol.-% Xenon.

12. Medikament für eine Verwendung nach einem der vorstehenden Ansprüche, **dadurch gekennzeichnet, dass** es dem Patienten ein oder mehrere Male pro Tag verabreicht wird.

13. Medikament für eine Verwendung nach einem der vorstehenden Ansprüche, **dadurch gekennzeichnet, dass** es dem Patienten während einer Inhalationsdauer von einigen Minuten bis einigen Stunden, insbesondere zwischen 15 Minuten und 4 Stunden, verabreicht wird.

## Claims

1. Gaseous medication containing xenon for a use via inhalation combined with at least one NMDA receptor antagonist in the form of a liquid or solid, for treating, slowing or preventing neurological deterioration consecutive to a neurodegenerative disease in a human patient.

2. Medication for a use according to the preceding claim, **characterised in that** it contains between 10 and 80 % by volume of xenon.

3. Medication for a use according to one of the preceding claims, **characterised in that** the NMDA receptor antagonist in the form of a solid, in particular in the form of a tablet or capsule.

4. Medication for a use according to one of the preceding claims, **characterised in that** the NMDA receptor antagonist is memantine or nitromemantine.

5. Medication for a use according to one of the preceding claims, **characterised in that** the neurodegenerative disease is Alzheimer's disease or Parkinson's disease, preferably Alzheimer's disease.

6. Medication for a use according to one of the preceding claims, **characterised in that** the daily dose of NMDA receptor antagonist administered to the patient is less than or equal to 20 mg/day.

7. Medication for a use according to one of the preceding claims, **characterised in that** the gaseous xenon is administered to the patient before, concomitantly or after administration of the NMDA receptor antagonist, preferably after administration of the NMDA receptor antagonist.

8. Medication for a use according to one of the preceding claims, **characterised in that** the neurological deterioration comprises an excessive intake of Ca²⁺ ions in one or more populations of vulnerable neurones of the patient.

9. Medication for a use according to one of the preceding claims, **characterised in that** it furthermore contains oxygen, preferably at least 21 % by volume of oxygen, preferably it is comprised of xenon and oxygen or of xenon, nitrogen and oxygen.

10. Medication for a use according to one of the preceding claims, **characterised in that** the patient is a human being over the age of 30 years old, in particular over the age of 50 years old.

11. Medication for a use according to one of the preceding claims, **characterised in that** it contains at least 20 % by volume of xenon and/or it contains less than 75 % by volume of xenon, preferably less than 60 % by volume of xenon.

12. Medication for a use according to one of the preceding claims, **characterised in that** it is administered to the patient, one or more times a day.

13. Medication for a use according to one of the preceding claims, **characterised in that** it is administered to the patient during a period of inhalation of a few minutes to a few hours, in particular between 15 minutes and 4 hours.
